(19)

Europäisches
Patentamt
European
Patent Office
Office européen
des brevets

(11) **EP 1 514 514 B1**

(12) **EUROPEAN PATENT SPECIFICATION**

(45) Date of publication and mention
of the grant of the patent:
**16.06.2010 Bulletin 2010/24**

(51) Int Cl.:
***A61B 5/107*** *(2006.01)*  ***A61B 5/053*** *(2006.01)*

(21) Application number: **04021223.5**

(22) Date of filing: **07.09.2004**

(54) **Impedance type thickness measurement device**

Impedanzvorrichtung für Dickenmessungen

Dispositif à impédance pour la mesure d'épaisseurs

(84) Designated Contracting States:
**DE FR GB**

(30) Priority: **11.09.2003 JP 2003319862**

(43) Date of publication of application:
**16.03.2005 Bulletin 2005/11**

(73) Proprietor: **TANITA CORPORATION
Tokyo (JP)**

(72) Inventor: **Izumi, Shuichi
Tokyo (JP)**

(74) Representative: **Müller-Boré & Partner
Patentanwälte
Grafinger Strasse 2
81671 München (DE)**

(56) References cited:
**EP-A- 1 329 191    US-A- 5 184 624**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## EP 1 514 514 B1

**Description**

<u>Background of the Invention:</u>

<u>Field of the Invention:</u>

The present invention relates to a measurement device for measuring the thickness (i.e. girth, radius or diameter) of a body part of a person under test by means of impedance measurement.

<u>Prior Art:</u>

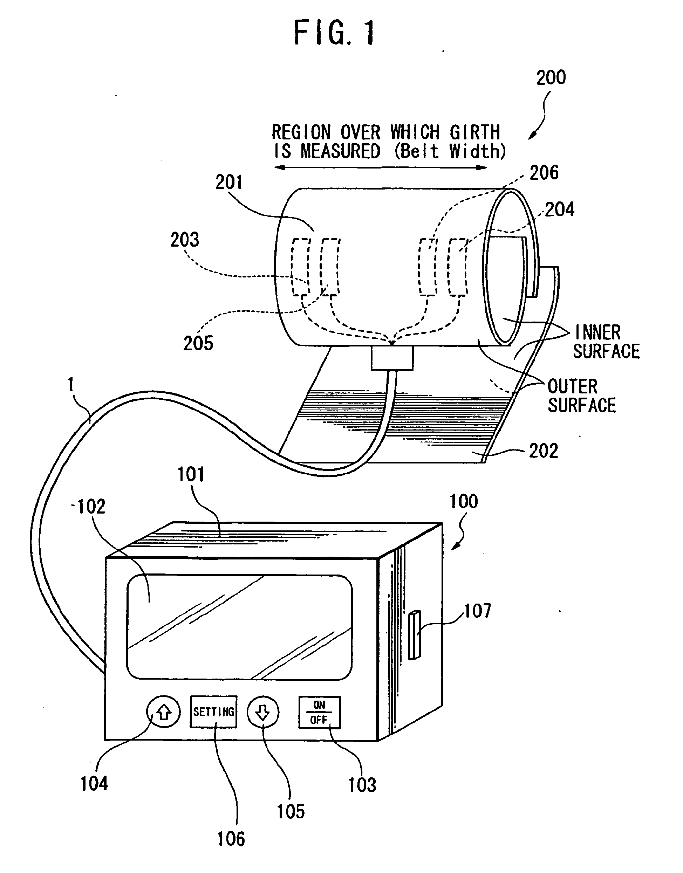

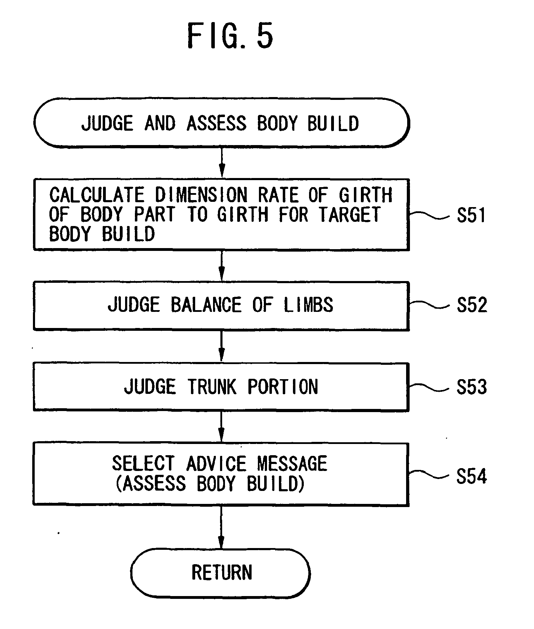

In the prior art, a kind of a tape measure has frequently been used for measuring the girth of a body part by wrapping it around the body part. Various types of the tape measures for measuring the girth of trunk portion have been developed, including, for example, a girth measurement unit as shown in Fig. 1 of Japanese Patent Laid-Open No. 2000-350727, an encoder type measure as shown in Fig. 5 of Japanese Patent Laid-Open No. 2002-191563, a waist measurement unit as shown in Fig. 1 of WO 01/076485, etc. Those tape measures are designed for measuring the girth of body part only over a limited narrower region thereof. For example, they could measure the girth of trunk portion over a narrower region thereof at the width of 1 to 2 cm.

There has also been known a measurement device for measuring the body part using the living body impedance measurement technique. One example of such living body measurement device can be found in Japanese Patent Laid-Open No. 2001- 178696, which is designed for measurement of body fat rate (internal composition) of each part (a right hand, a left hand, a right foot, a left foot and a trunk) of the body.

The following patent documents are associated with the present invention:

Patent Document 1: Japanese Patent Laid-Open No. 2000-350727;
Patent Document 2: Japanese Patent Laid-Open No. 2002-191563;
Patent Document 3: WO 01/076485; and
Patent Document 4: Japanese Patent Laid-Open No. 2001-178696.

According to the prior art method using a tape measure, as described above, if it is desired to measure the girth of body part over a wider region, it is, of course, necessary to repeatedly measure it for a plurality of narrower regions and to average the measured values. For example, if it is desired to measure the girth of trunk portion for a region extending from adjacent the pit of stomach to adjacent the waist, it is necessary to measure it at least at the positions adjacent the pit of stomach, adjacent the navel and adjacent the waist, and then, to average the measured values.

Furthermore, the living body measurement device using living body impedance measurement technique, as described above, simply acts to measure the internal composition of the body part and to display the result of measurement. That is to say, it is not configured to measure the external geometry of the body part and to judge and assess the result of measurement.

EP 1 329 191 A1 discloses a health care administration device in which a waist size or hip size of an examinee can be computed using the body fat percentage of the examinee.

US 5 184 624 discloses a method of determining the external shape of a body preparatory to obtaining tomographic images of it. For the determination of the external shape, a plurality of electrodes defining a plane are connected to the body surface and subsequently the distance between pairs of respectively adjacent electrodes is determined to fix the scale of the boundary.

It is the object of the invention to provide an improved impedance type thickness measurement device.

This object is fulfilled by an impedance type thickness measurement device having the features disclosed in claim 1. Preferred embodiments are defined in the dependent subclaims.

According to the present invention there is provided of an impedance type thickness measurement device that has capability of measurement of the thickness (i.e. girth, radius or diameter) of a body part over a wide region thereof, without any difficulty.

Moreover, there is provided an impedance type thickness measurement device that has capability of judgment and assessment for the thickness (i.e. girth, radius or diameter) of a body part over a wide region thereof, which is very useful for a person under test.

<u>Summary of the Invention:</u>

To attain those objects the present invention provides, in one aspect thereof, an impedance type thickness measurement device according to claim 1, comprising: an impedance measurement unit; and an arithmetic unit, wherein

said impedance measurement unit measures the impedance of a part of a body to be measured, and
said arithmetic unit calculates the actually measured thickness of the part of the body, based on the impedance measured by said impedance measurement unit.

A display unit displays a body model made up of a plurality of body parts to be measured independently and displays which body part is going to be measured, by marking the relevant part on the body model, when the impedance is measured by said impedance measurement unit.

The impedance type thickness measurement device further comprises a storage unit and a judgment and assessment unit, said storage unit stores body build judgment and assessment information for exhibiting the relation between the thickness of the body part for some target body build and the actually measured thickness of the body part, and said judgment and assessment unit judges and assesses the body build on the basis of the actually measured thickness calculated by said arithmetic unit, with reference to the body build judgment and assessment information stored in said storage unit.

According to one embodiment of the present invention, said display unit displays the actually measured thickness of the part of the body calculated by said arithmetic unit.

According to yet further embodiment of the present invention said impedance measurement unit includes a personal information input unit by which personal information is entered,
said storage unit stores the body build judgment and assessment information including: a target body build selection table for exhibiting the relation between the personal information and the thickness of the body part for the target body build; a dimension rate calculation formula for calculating the dimension rate of the actually measured thickness of the body part to the thickness of the body part for the target body build; and a body build judgment and assessment table for exhibiting the relation between the dimension rate calculated, acceptability of the body part depending on the dimension rate, and some advice about the acceptability of the body part,
said judgment and assessment unit conducts judgment and assessment for the body build by the steps of: selecting the thickness of the body part for the target body build corresponding to the personal information entered by said personal information input unit, with reference to the target body build selection table stored in the storage unit; calculating the dimension rate by substituting the selected thickness of the body part for the target body build and the actually measured thickness of the body part provided by said arithmetic unit for said dimension rate calculation formula stored in the storage unit; judging the acceptability of the body part measured by said impedance measurement unit, based on the dimension rate thus calculated, with reference to the body build judgment and assessment table stored in the storage unit; and selecting some advice information depending on the acceptability of the body part.

Said display unit further displays the result of judgment and assessment for body build provided by said judgment and assessment unit.

According to yet further embodiment of the present invention said display unit displays a body part having the actually measured thickness that is outside the target thickness range, as determined by said judgment and assessment unit, by marking the relevant part on the body model.

According to yet further embodiment of the present invention said body model is formed such that each target body build can be imaged.

According to yet further embodiment of the present invention said impedance measurement unit includes current supplying electrodes and measurement electrodes all made contact to the body part to be measured.

According to yet further embodiment of the present invention said current supplying electrodes and said measurement electrodes are mounted on a belt that is wrapped around the body part to be measured.

According to yet further embodiment of the present invention said belt includes a main belt on which said current supplying electrodes and said measurement electrodes are mounted and an extension belt adapted for connection with the main belt as necessary.

According to yet further embodiment of the present invention one pair of current supplying and measurement electrodes and another pair of current supplying and measurement electrodes are arranged on said belt in such manner that each pair is positioned near each side edge of the belt and is aligned to the other pair in the width direction of the belt.

According to yet further embodiment of the present invention said current supplying electrodes and said measurement electrodes are mounted on a contact plate that is abut against the body part to be measured.

According to yet further embodiment of the present invention one pair of current supplying and measurement electrodes and another pair of current supplying and measurement electrodes are arranged on said contact plate in such manner that each pair is positioned near each side edge of the contact plate and is aligned to the other pair in the width direction of the contact plate.

According to yet further embodiment of the present invention said measurement electrodes are disposed between said current supplying electrodes.

According to yet further embodiment of the present invention the thickness of the body part is any one of girth, radius and diameter of the body part.

An impedance type thickness measurement device according to the present invention can measure the thickness (i.e. girth, radius or diameter) of a body part over a wide region thereof, without any difficulty.

Furthermore, the impedance type thickness measurement device can judge and assess the thickness (i.e. girth, radius or diameter) of a body part over a wide region thereof, which is advantageous in that a person under test can get the information useful for health care and for maintaining the target body build.

Brief Description of the Drawings:

Now, the present invention will be described in more detail with reference to the accompanying drawings, in which:

Fig. 1 is an external view illustrating an entire configuration of an impedance type thickness measurement device according to one embodiment of the present invention;
Fig. 2 is a block diagram for explanation of internal construction of the device in Fig. 1;
Fig. 3 is a schematic diagram for explaining how to use the device in Fig. 1;
Fig. 4 is a main flow chart illustrating a measurement sequence of the device in Fig. 1;
Fig. 5 is a subroutine flow chart illustrating the measurement sequence of the device in Fig. 1;
Fig. 6 is a view illustrating a body build judgment and assessment table exhibiting the relation between dimension rate, acceptability of body part and advice;
Fig. 7 is a display screen for entering personal information;
Fig. 8 is a display screen for selecting target body build;
Fig. 9 is a display screen for confirming input items and for starting measurement;
Fig. 10 is a display screen for displaying measurement result and advice;
Fig. 11 is a schematic diagram illustrating modified embodiment of the present invention;
Fig. 12 is a view, similar to Fig. 8, but illustrating the display screen according to the modified embodiment in Fig. 11;
Fig. 13 is a view, similar to Fig. 9, but illustrating the display screen according to the modified embodiment in Fig. 11;
Fig. 14 is a view, similar to Fig. 10, but illustrating the display screen according to the modified embodiment in Fig. 11;
Fig, 15 is a view illustrating a detector of the impedance type thickness measurement device according to another embodiment;
Fig, 16 is a view illustrating a detector of the impedance type thickness measurement device according to further embodiment; and
Fig. 17 is a view illustrating target body build selection table exhibiting the relation between personal information and thickness of body part for target body build.

Description of the Preferred Embodiments:

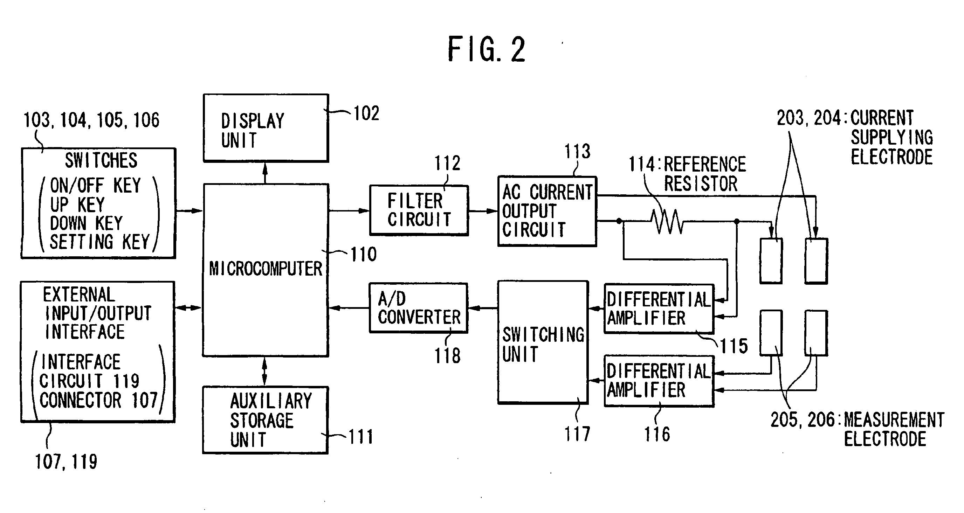

Fig. 1 is an external view illustrating an entire configuration of an impedance type thickness measurement device according to one embodiment of the present invention, and Fig. 2 is a block diagram for explanation of internal construction of the device in Fig. 1. As is apparent in Fig. 1, the impedance type thickness measurement device according to this embodiment comprises a main frame 100 and a detector 200 connected to the main frame 100 via a cord 1.

The main frame 100 includes a casing 101 having various types of components mounted therein, which are described later in more detail. On the external surface of the casing 100 there are provided a display unit 102; various types of keys such as an ON/OFF key 103, an UP key 104, a DOWN key 105 and a setting key 106; and a connector 107 forming a part of an external input/output interface.

Referring to Fig. 2, included in the casing 101 and mounted on a board unit are: a microcomputer 110; an auxiliary storage unit 111; a filter circuit 112; an AC current output circuit 113; a reference resistor 114; differential amplifiers 115 and 116; a switching unit 117; an A/D converter 118; and an interface circuit 119 forming a part of the external input/output interface.

On the other hand, as shown in Fig. 1, the detector 200 includes a main belt 201, an extension belt 202 adapted for connection with an end of the main belt 210 as necessary, and current supplying electrodes 203, 204 and measurement electrodes 205, 206 all positioned on the main belt 201. The detector 200 is provided for detecting the impedance of a body part.

The current supplying electrodes 203, 204 and the measurement electrodes 205, 206 are arranged on the main belt 201 in such manner that they are positioned at the side near the body part (i.e. at the inner side of the main belt) when it is wrapped around the body part and they are aligned to each other in the width direction of the main belt. Furthermore, the current supplying electrodes 203, 204 and the measurement electrodes 205, 206 are arranged in such manner that the measurement electrodes 205, 206 are present between the current supplying electrodes 203, 204, as shown in Fig. 1. In addition, one pair of the current supplying electrode 203 and the measurement electrode 205 and another pair of the current supplying electrode 204 and the measurement electrode 206 are disposed adjacent the side edges of the main belt 201 with some spacing between each pair of electrodes. The current supplying electrodes 203, 204 are provided for applying the electric current to the body part to be measured and the measurement electrodes 205, 206 are provided for detecting any voltage produced on the body part by application of the electric current.

The main belt 201 and the extension belt 202 are configured so that the inner surface at one end is engaged with the outer surface at the other end when they are wrapped around the body part in the same manner as a cuff of the prior art sphygmomanometer. Furthermore, the main belt 201 and the extension belt 202 have such width that they can surround the body part over the zone of as much wider as possible when they are wrapped around the body part.

As shown in Fig. 1, the cord 1 is provided in such manner that one end thereof is connected to the current supplying electrodes 203, 204 and the measurement electrodes 205, 206 and the other end thereof is connected to the differential amplifiers 115 and 116, the AC current output circuit 113 and the reference resistor 114 on the board unit within the casing 101.

Next, each of the components in the present device will be described in more detail. The display unit 102 displays the followings: input items such as personal information, target body build, etc.; parts of the body to be measured; result of measurement such as girth, etc.; advice messages; and the like.

The ON/OFF key 103 is provided to power ON or OFF the present device.

The UP key 104 and the Down key 105 are provided to increase or decrease the numerical value entered and to move the cursor upwardly or downwardly.

The setting key 106 is provided to decide the numerical value selected by the UP key 104 and the Down key 105 and the position of the cursor.

The connector 107 is used for communication with the external devices.

The microcomputer 110 includes a CPU, a ROM, a RAM, a timer, an I/O port, etc., for performing the control for input of personal information and target body build, measurement of living body impedance, calculation of girth, etc., judgment for body build (balance of limbs, trunk portion) and assessment for body build (selection of advice message). In particular, the ROM stores: control and calculation programs; calculation formulae for calculating girth, dimension rate, etc.; constants to be substituted for the calculation formulae; target body build selection tables; body build judgment and assessment tables; etc. The RAM temporary stores: result of calculation; programs retrieved from some external units; input data items such as personal information and target body build; etc. The target body build selection table exhibits the relation between the personal information and the thickness of body part for the target body build. The body build judgment and assessment table exhibits the relation between the dimension rate for actually measured thickness, acceptability of the body part relative to the dimension rate, and advice about the acceptability of the body part.

The interface circuit 119 is provided for sending and receiving signals to and from the external devices via the connector 107.

The auxiliary storage unit 111 stores the input data items such as personal information and target body build so that they can be updated.

The filter circuit 112 acts to form the signal output from the microcomputer 110 into a signal to be applied to the living body.

The AC current output circuit 113 receives the signal from the filter circuit 112 and produces the fixed RMS signal.

The reference resistor 114 has one end connected to the output of the AC current output circuit 113 and acts to compensate for any effect on the impedance due to change in fixed current from the AC current output circuit 113.

One differential amplifier 115 amplifies the voltage across both terminals of the reference resistor 114 and the other differential amplifier 116 amplifies the voltage detected by the measurement electrodes 205 and 206.

The switching unit 117 selects any one of the outputs of the differential amplifiers 115 and 116 under the control of the microcomputer 110.

The A/D converter 118 converts the analog signal from the switching unit 117 into the digital signal which is then output to the microcomputer 110.

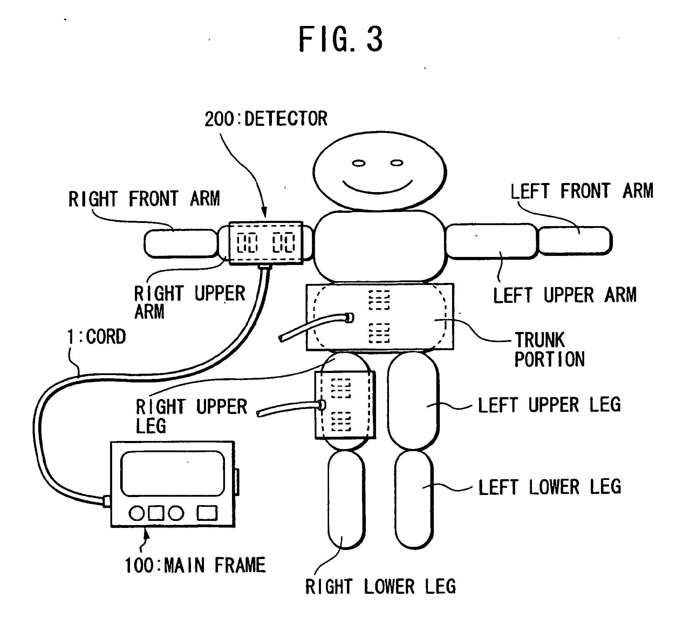

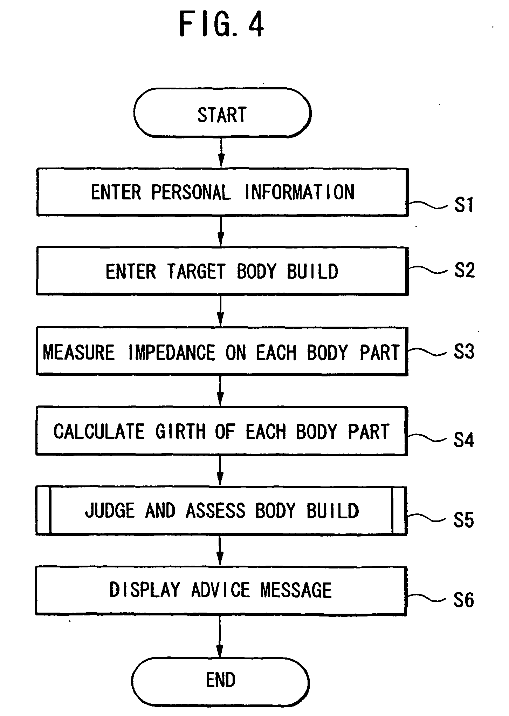

Now, operation and function of the impedance type thickness measurement device having the configuration according to the above-mentioned embodiment will be described in more detail. Fig. 3 is a schematic diagram for explaining how to use the present device, Fig. 4 is a main flow chart illustrating a measurement sequence of the present device, and Fig. 5 is a subroutine flow chart.

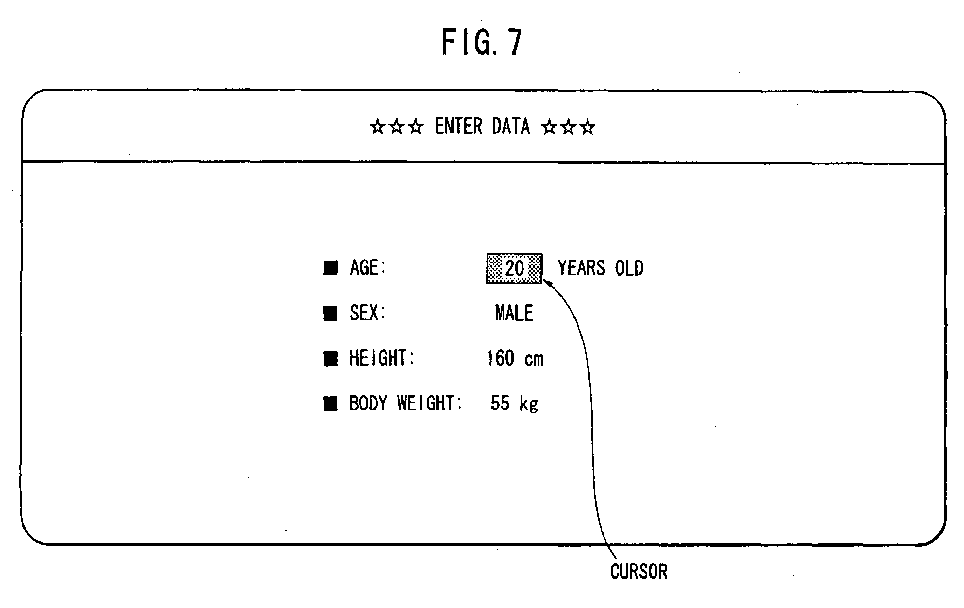

Prior to start the measurement the ON/OFF key 103 on the casing 101 of the main frame 100 is turned ON to put the present device in operation mode and the display unit 102 display an input screen for entering the personal information, as shown in Fig. 7. Then, at step S1 of the main flow chart of Fig. 4 wherein the personal information is entered, a user sets the personal information such as age, sex, height, and body weight on the screen, as shown in Fig. 7, using the UP key 104, the DOWN key 105 and the setting key 106. Referring to Fig. 7, the numerical values or characters pointed by the cursor are changed by the UP key 104 and the DOWN key 105, and are decided by depressing the setting key 106. The decided personal information is stored in the auxiliary storage unit 111.

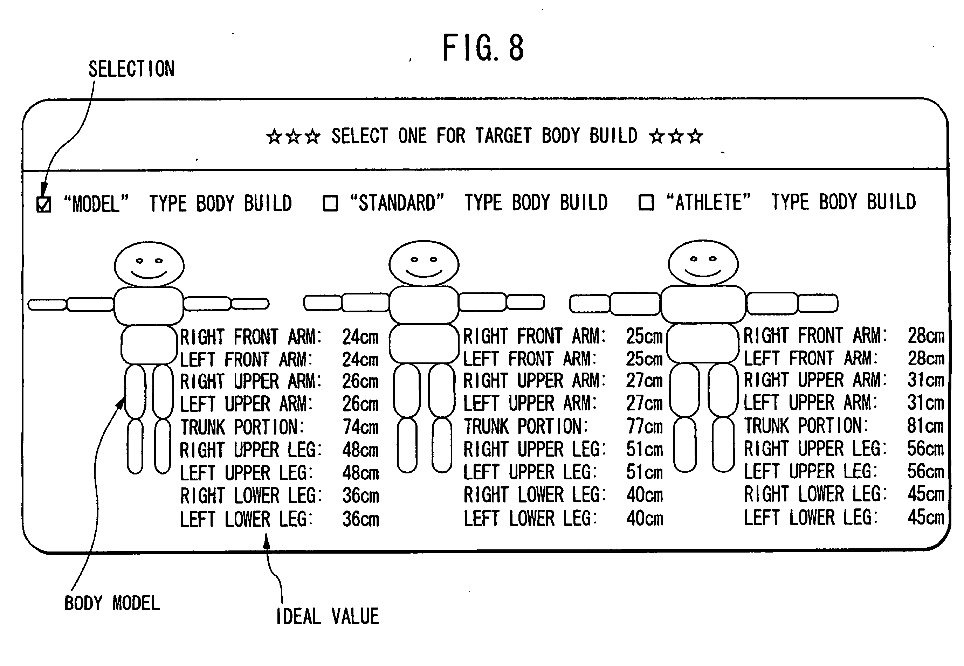

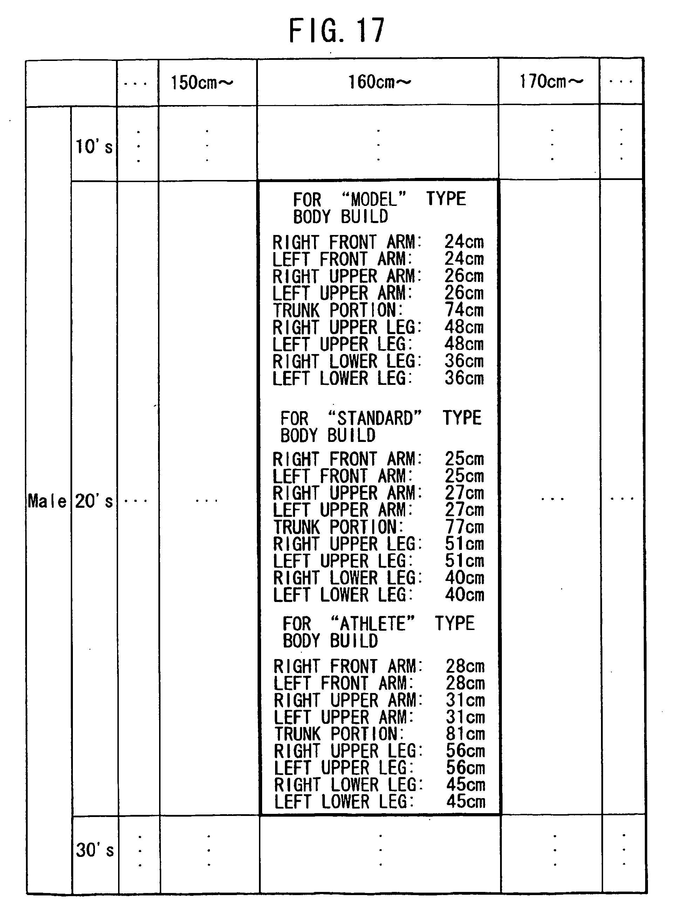

After the numerical value for body weight is decided, the thickness of the body part for the target body build corresponding to the decided personal information is selected, with reference to the target body build selection table stored in the ROM, as shown in Fig. 17 (for example, the data encircled by thick-line rectangle in Fig. 17 may be selected for the personal information exemplified in Fig. 7). Then, the display unit 102 displays a screen for entering the target body build, as shown in Fig. 8. More specifically, the display unit displays: the body builds to be selected such as "MODEL" type, "STANDARD" type, and "ATHLETE" type; body models each making possible to imagine each of target body builds (the body model has the body parts at different width for each of target body builds); and ideal values for body parts for each of the target body builds (i.e. average values for a target person for each of the target body builds). Then, at step S2 of the main flow chart of Fig. 4, the user select the target body build on the screen, as shown in Fig. 8. In particular, at step S2, the UP key 104 and the DOWN key 105 are used to select any one of the body builds by filling the corresponding square mark "□". Thereafter, the setting key 106 is depressed to decide the body build. The data of decided target body build is stored in the auxiliary storage unit 111.

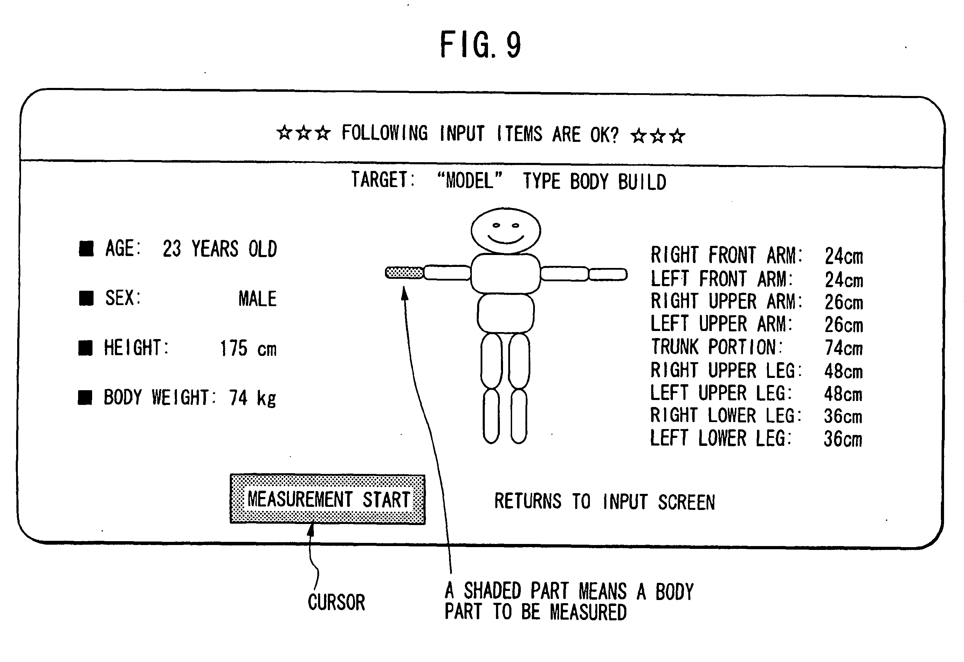

Thereafter, the display unit 102 displays the information already entered and decided and the body part to be measured on the screen, as shown in Fig. 9. In this case, each time when the UP key 104 or the DOWN key 105 is depressed, the cursor is moved from the position ''MEASUREMENT START" to the position "RETURNS TO INPUT SCREEN" and vice-versa. If there is an error found and it is desired to correct it then the UP key 104 or the DOWN key 105 is depressed to move the cursor from ''MEASUREMENT START" to "RETURNS TO INPUT SCREEN". Thereafter, the setting key 106 is depressed to return to the personal information input screen, as shown in Fig. 7, where it is possible to repeat the process sequence from step S1.

On the other hand, when measurement of the impedance on each body part is conducted at step S3 in Fig. 4, initially, the main belt 201 of the detector 200 (with the extension belt 202 connected thereto as necessary) is worn to the measured body part of the user corresponding to the body part (the shaded part) of the body model on the screen in Fig. 9. Then, while the cursor is positioned at ''MEASUREMENT START" the setting key 106 is depressed to start measurement of the impedance. After completion of measurement of impedance on a right front arm, the measured body part (the shaded part) of the body model on the screen in Fig. 9 is switched from the right front arm to a right upper arm. Accordingly, the main belt 201 of the detector 200 is worn to that body part of the user and the setting key 106 is depressed to continue measurement of the impedance. Thereafter, in the same manner, measurement of the impedance is conducted in the sequence of a left front arm, a left upper arm, a right lower leg, a left lower leg, a right upper leg, a left upper leg, and a trunk portion. It is noted, here, that when measurement is conducted on the left and right front arms, the left and right upper arms, and the left and right lower legs then only the main belt 201 is wrapped around without the extension belt 202 connected thereto. However, when measurement is conducted on the left and right upper legs and on the trunk portion then the main belt 201 is wrapped around with the extension belt 202 connected thereto by engaging the inner surface of the extension belt 202 with the outer surface of the main belt 201.

After completion of measurement of the impedance on all the body parts, at step S4 in the main flow chart of Fig. 4, the microcomputer 110 calculates the girth of each of the body parts using the following calculation formula (1). At first, the girth of the right front arm is calculated by substituting the measured impedance of the right front arm, the resistivity of the right front arm already stored in the ROM, the distance between the measurement electrodes, and the ratio of the circumference of a circle to its diameter for the calculation formula (1). Then, the girth of the right upper arm is calculated by substituting the measured impedance of the right upper arm, the resistivity of the right upper arm already stored in the ROM, the distance between the measurement electrodes, and the ratio of the circumference of a circle to its diameter for the calculation formula (1). In the same manner, the girth of the left front arm, the left upper arm, the right lower leg, the left lower leg, the right upper leg, the left upper leg, and the trunk portion is successively calculated.

$$Cm = 2\sqrt{\frac{\rho L \pi}{Z}} \qquad (1)$$

where Cm: Girth of the body part;
$\rho$ : Resistivity of the body part;
$\pi$ : Ratio of the circumference of a circle to its diameter;
L: Distance between the measurement electrodes; and
Z: Impedance of the body part.

It is noted, here, that the resistivity " $\rho$ " is a factor (or a constant) derived on the basis of the data such as girth of body part, impedance of body part and distance between measurement electrodes, as collected from many people for each of target body builds. The distance between measurement electrodes is one that is measured while they are actually arranged on the main belt 201 of the detector 200.

Then, at step S5 in the main flow chart of Fig. 4, the microcomputer 110 performs judgment and assessment for body build. Operation at step S5 is processed, as shown in the subroutine flow chart of Fig. 5. Initially, at step S51, the dimension rate of the girth of each body part calculated to the girth of each body part for the target body build selected is calculated by the following formula (2):

$$Pm = \frac{Cm - Cs}{Cs} \times 100 \qquad (2)$$

where Pm: Dimension rate; and
Cs: Girth of body part for target body build.

Then, at steps S52 and S53, acceptability of the body part is judged on the basis of the dimension rate calculated in this way. More particularly, at step S52, balance of limbs is judged by determining whether the girth of each limb calculated is deviated within $\pm 3\%$ of the girth of each limb for target body build selected. In this example, it is assumed that the right front arm plus the right upper arm becomes a right upper limb; the left front arm plus the left upper arm becomes a left upper limb; the right lower leg plus right upper leg becomes a right lower limb; the left lower leg plus left upper leg becomes a left lower limb; and the right upper limb plus the left upper limb plus right lower limb plus the left lower limb becomes four limbs.

At step S53 it is determined whether the girth of trunk portion calculated is deviated within $\pm 3\%$ of the girth of trunk portion for target body build selected.

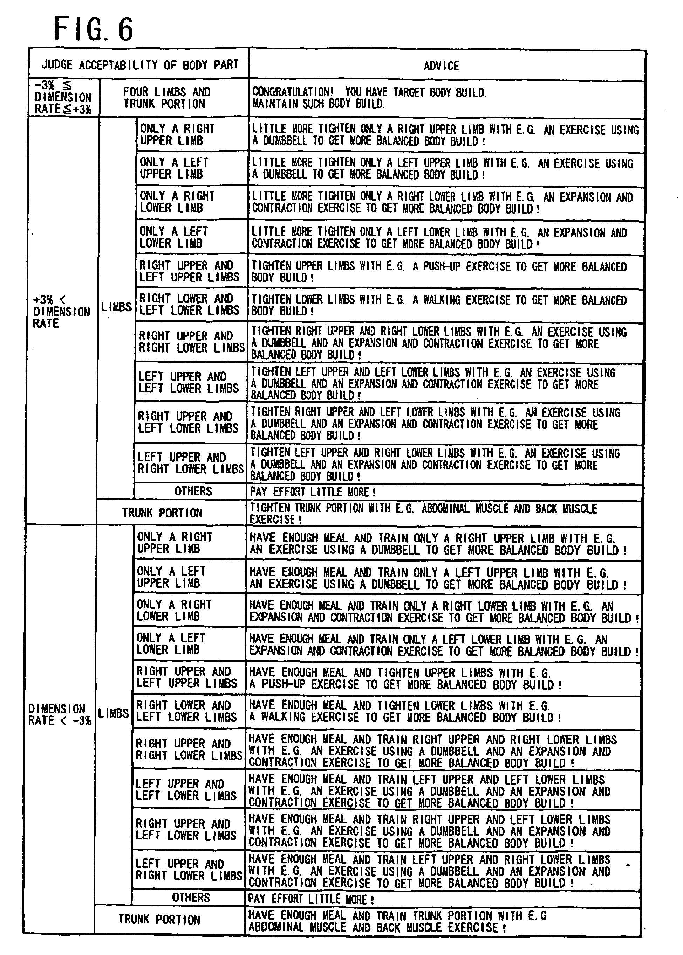

Then, at step S54, the assessment is performed by selecting some advice message in view of the balance of limbs and judgment for trunk portion (or judgment for acceptability of the body part) at steps S52 and S53, with reference to the body build judgment and assessment table, as shown in Fig. 6, which table is stored in the ROM. For example, if the girth of right lower limb calculated is deviated over $\pm 3\%$ of the girth of right lower limb for target body build selected, but the girth of each of the other limbs calculated is deviated within $\pm 3\%$ of the girth of each of the other limbs for target body build selected, an advice message "Little more tighten only a right lower limb with e.g. expansion and contraction exercise to get more balanced body build ! " is selected in view of judgment for balance of limbs. Furthermore, if the girth of trunk portion calculated is deviated over +3% of the girth of trunk portion for target body build selected, an advice message "Pay effort little more ! " is selected.

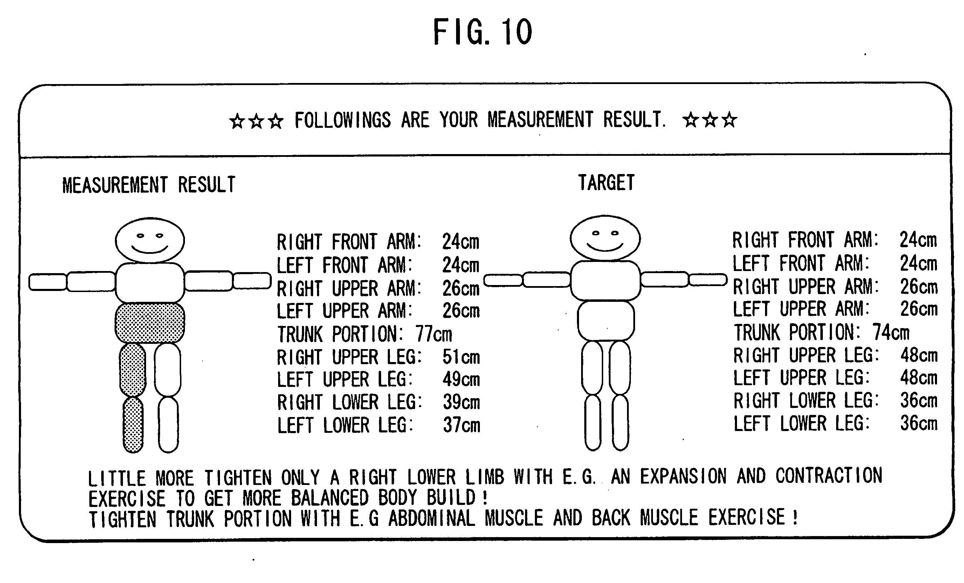

After selection of the advice message at step S54 the subroutine in Fig. 5 returns to the main routine in Fig. 4 where at step S6 the display unit 102 displays the girth of each of limbs calculated and the advice message selected on the screen thereof, as shown in Fig. 10. Then, operation of the present device is terminated.

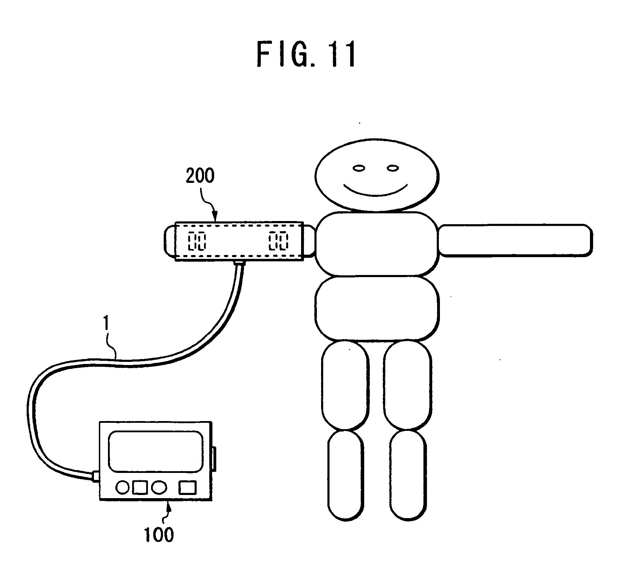

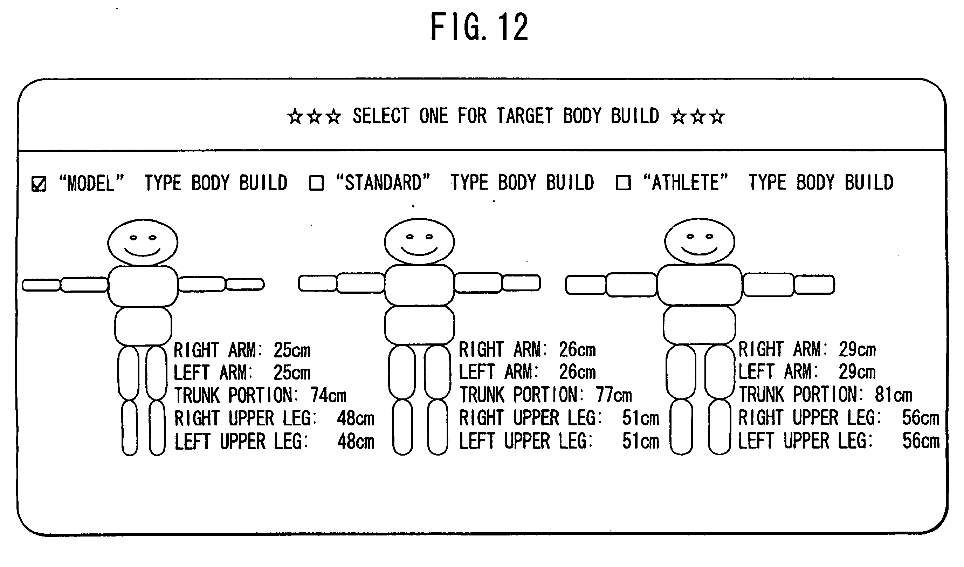

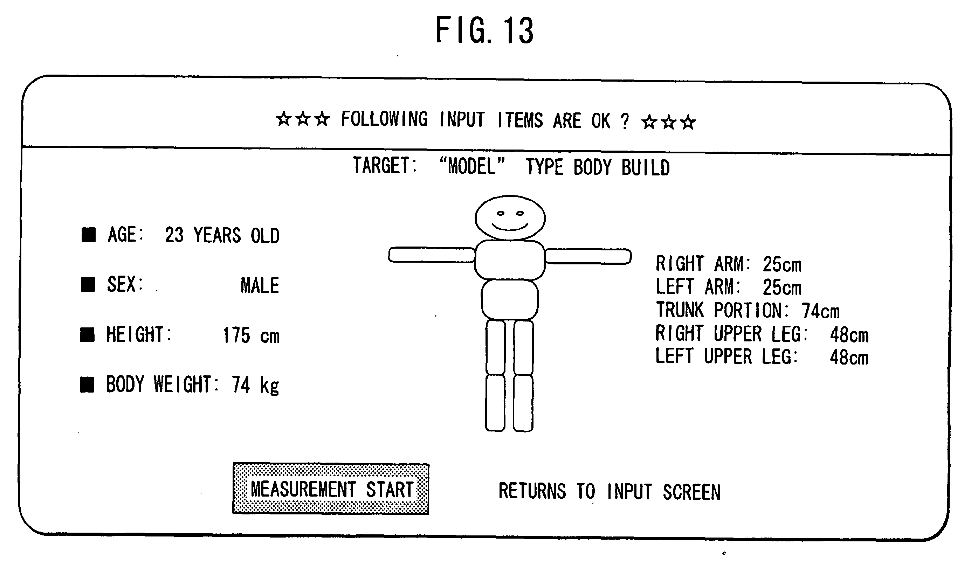

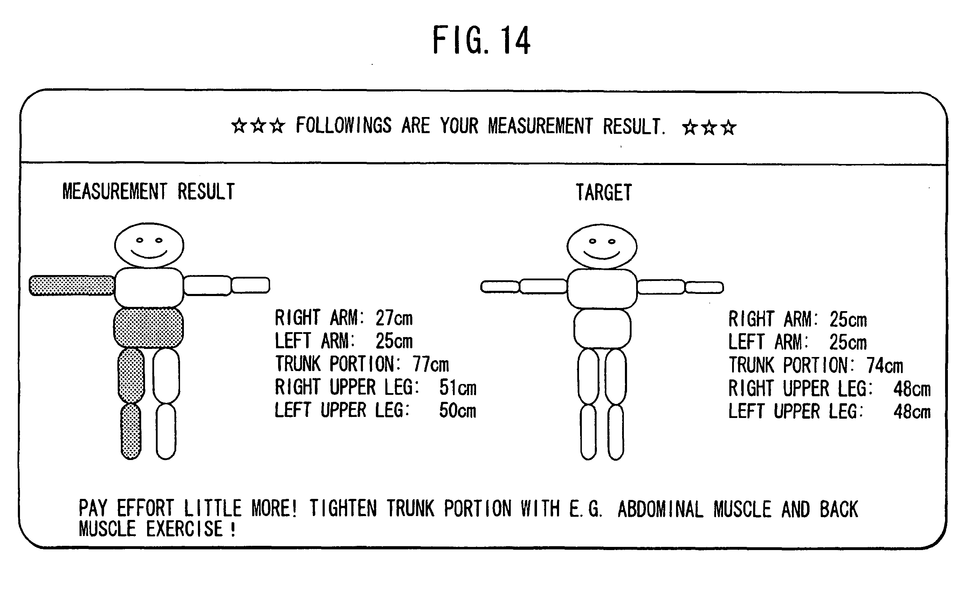

In the embodiment as described above, measurement of the impedance has been performed on the right front arm, right upper arm, left front arm, left upper arm, right lower leg, left lower leg, right upper leg, left upper leg and trunk portion. However, the present invention is not limited to such embodiment. For example, measurement of the impedance may be performed on a right upper limb (i.e. a right arm) made up of the right front arm and the right upper arm, and a left upper limb (i.e. a left arm) made up of the left front arm and the left upper arm. In such alternative embodiment the main belt of the detector 200 has such width that spans from adjacent a wrist of the front arm to adjacent a shoulder of the upper arm and the electrodes are positioned near the wrist and the shoulder, as shown in Fig. 11. Furthermore, the display unit 102 displays the information on the screen, as shown in Figs. 12 to 14.

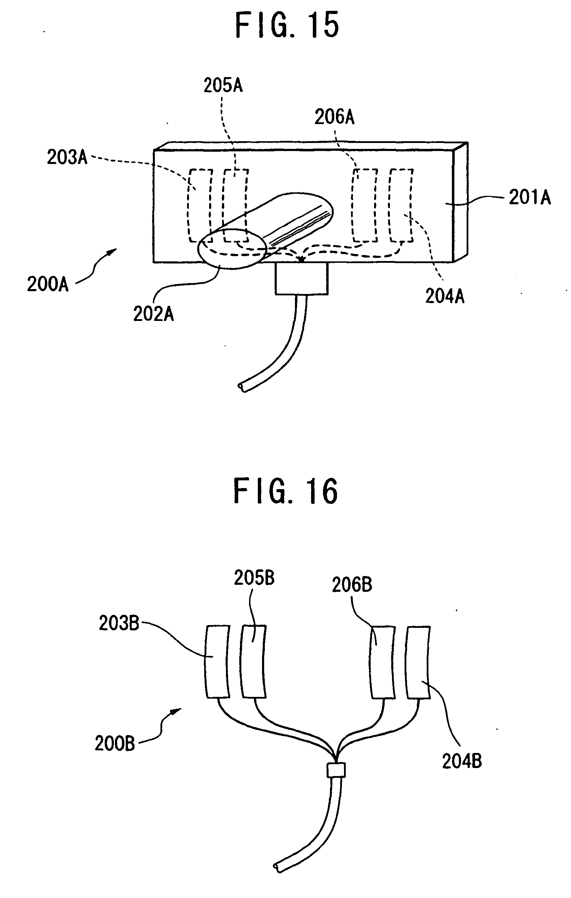

In the above-mentioned embodiment, the detector has been described as being wrapping type, similar to the prior art sphygmomanometer, made up of the main belt and the extension belt. However, it may be configured in another way, as shown in Fig. 15 or Fig. 16. Referring to Fig. 15, a detector 200A includes a contact plate 201A, a grip 202A mounted to the contact plate 201A, and current supplying electrodes 203A, 204A and measurement electrodes 205A, 206A arranged on one surface of the contact plate 201A. Referring to Fig. 16, a detector 200B includes current supplying electrodes 203B, 204B and measurement electrodes 205B, 206B which are not fixed and separated from each other.

The impedance type thickness measurement device in the embodiment as above has been described especially for the case where the thickness is girth of body part. However, the thickness may be radius or diameter of body part. For example, for the case where the thickness is radius of body part, at step S4, the radius of body part is calculated using the following formula (3) already stored in the ROM. Then, the dimension rate is calculated using the following formula (4), and at step 5, it is determined whether the radius of each limb calculated is deviated within $\pm 3\%$ of the radius of each limb for target body build selected. On the other hand, for the case where the thickness is diameter of body part, at step S4, the diameter of body part is calculated using the following formula (5) already stored in the ROM. Then, the dimension rate is calculated using the following formula (6), and at step 5, it is determined whether the diameter of each limb calculated is deviated within $\pm 3\%$ of the diameter of each limb for target body build selected. It is noted, here, that for case where the thickness is radius or diameter of body part the ideal value and measured value for each body part displayed on the screen in Figs. 8, 9, 10, 12, 13 and 14 are those for radius or diameter.

$$Rm = \sqrt{\frac{\rho L}{Z\pi}} \qquad\qquad (3)$$

where Rm: Radius

ρ : Resistivity of the body part;

π: Ratio of the circumference of a circle to its diameter;

L: Distance between the measurement electrodes; and

Z: Impedance of the body part.

$$P = \frac{Rm - Rs}{Rs} \times 100 \qquad\qquad (4)$$

where P: Dimension rate; and

Rs: Radius of body part for target body build.

$$Dm = 2\sqrt{\frac{\rho L}{Z\pi}} \qquad\qquad (5)$$

where Dm: Diameter

ρ : Resistivity of the body part;

π: Ratio of the circumference of a circle to its diameter;

L: Distance between the measurement electrodes; and

Z: Impedance of the body part.

$$P = \frac{Dm - Ds}{Ds} \times 100 \qquad\qquad (6)$$

where P: Dimension rate; and

Ds: Diameter of body part for target body build.

Industrial Availability:

The present invention can measure the thickness of a body part over a wide region thereof, without any difficulty, and can judge and assess the thickness measured in this way, which provides the information useful for a user to conduct health care and to maintain the target body build. Accordingly, the present invention can find application to beauty and health care industry.

**Claims**

1.  An impedance type thickness measurement device, comprising:

    an impedance measurement unit (104, 105, 106, 110, 112, 113, 114, 115, 116, 117, 118, 200);
    an arithmetic unit (110);
    a storage unit (110, 111); and
    a judgment and assessment unit (110), wherein
    said impedance measurement unit (104, 105, 106, 110, 112, 113, 114, 115, 116, 117, 118, 200) is adapted to
    measure the impedance of a part of a body to be measured;

said arithmetic unit (110) is adapted to calculate a thickness of the part of the body, based on the impedance measured by said impedance measurement unit (104, 105, 106, 110, 112, 113, 114, 115, 116, 117, 118, 200); said storage unit (110, 111) is adapted to store body build judgment and assessment information for exhibiting the relation between the thickness of the body part for a target body build and the calculated thickness of the body part; and

said judgment and assessment unit (110) is adapted to judge and assess the body build on the basis of the thickness calculated by said arithmetic unit (110), with reference to the body build judgment and assessment information stored in said storage unit (110, 111)

**characterized in that** it further comprises

a display unit (102), said display unit (102) being adapted to display a body model made up of a plurality of body parts to be measured independently and display the result of judgment and assessment for body build performed by said judgment and assessment unit (110), wherein said display unit (102) is adapted to display a body part having the calculated thickness that is out-side a target thickness range, as determined by said judgment and assessment unit (110), by marking the relevant part on the body model.

2. An impedance type thickness measurement device according to claim 1 in which said impedance measurement unit (104, 105, 106, 110, 112, 113, 114, 115, 116, 117, 118, 200) includes a personal information input unit (104, 105, 106) by which personal information is entered,

said storage unit (110, 111) is adapted to store the body build judgment and assessment information including: a target body build selection table for exhibiting the relation between the personal information and the thickness of the body part for the target body build; a dimension rate calculation formula for calculating the dimension rate of the calculated thickness of the body part to the thickness of the body part for the target body build; and a body build judgment and assessment table for exhibiting the relation between the dimension rate calculated, acceptability of the body part depending on the dimension rate, and some advice about the acceptability of the body part,

said judgment and assessment unit (110) is adapted to conduct judgment and assessment for the body build by the steps of: selecting the thickness of the body part for the target body build corresponding to the personal information entered by said personal information input unit, with reference to the target body build selection table stored in the storage unit (110, 111); calculating the dimension rate by substituting the selected thickness of the body part for the target body build and the calculated thickness of the body part provided by said arithmetic unit (110) for said dimension rate calculation formula stored in the storage unit (110, 111); judging the acceptability of the body part measured by said impedance measurement unit (104, 105, 106, 110, 112, 113, 114, 115, 116, 117, 118, 200), based on the dimension rate thus calculated, with reference to the body build judgment and assessment table stored in the storage unit (110, 111); and selecting some advice information depending on the acceptability of the body part.

3. An impedance type thickness measurement device according to claim 1 in which said body model is formed such that each target body build can be imaged.

4. An impedance type thickness measurement device according to any one of claims 2 to 3 in which the thickness of the body part is the girth of the body part.

5. An impedance type thickness measurement device according to claim 4 in which said dimension rate calculation formula is

$$Pm = (Cm - Cs) \times 100/Cs$$

where Pm: Dimension rate;
Cm: the calculated Girth of the body part; and
Cs: Girth of body part for target body build
and;
said judgment and assessment unit (110) is adapted to judge the acceptability of the body part by determining whether the dimension rate calculated by said formula $Pm = (Cm - Cs) \times 100/Cs$ is deviated within $\pm$ 3%.

6. An impedance type thickness measurement device according to any one of claims 2 to 3 in which the thickness of the body part is the radius of the body part.

7. An impedance type thickness measurement device according to claim 6 in which said dimension rate calculation

formula is

$$P = (Rm - Rs) \times 100/Rs$$

where P: Dimension rate;
Rm: the calculated Radius of the body part; and
Rs: Radius of body part for target body build
and;
said judgment and assessment unit (110) is adapted to judge the acceptability of the body part by determining whether the dimension rate calculated by said formula P = (Rm - Rs) x 100/Rs is deviated within ± 3%.

8.  An impedance type thickness measurement device according to any one of claims 2 to 3 in which the thickness of the body part is the diameter of the body part.

9.  An impedance type thickness measurement device according to claim 8 in which said dimension rate calculation formula is

$$P = (Dm - Ds) \times 100/Ds$$

where P: Dimension rate;
Dm: the calculated Diameter of the body part; and
Ds: Diameter of body part for target body build
and;
said judgment and assessment unit (110) is adapted to judge the acceptability of the body part by determining whether the dimension rate calculated by said P = (Dm - Ds) x 100/Ds is deviated within ± 3%.

10. An impedance type thickness measurement device according to any one of claims 1 to 9 in which said impedance measurement unit (104, 105, 106, 110, 112, 113, 114, 115, 116, 117, 118, 200) includes current supplying electrodes (203, 204) and measurement electrodes (205, 206) adapted to make contact to the body part to be measured and comprises a main belt (201) and an extension belt (202) that are adapted to be wrapped around the body part to be measured.

11. An impedance type thickness measurement device according to claim 10 in which one pair (203, 205) of current supplying and measurement electrodes and another pair (204, 206) of current supplying and measurement electrodes are arranged on said main belt (201) in such manner that each pair (203, 205; 204, 206) is positioned near each side edge of the main belt (201) and is aligned to the other pair in the width direction of the belt.

12. An impedance type thickness measurement device according to any one of claims 1 to 9 in which said impedance measurement unit (104, 105, 106, 110, 112, 113, 114, 115, 116, 117, 118, 200) includes current supplying electrodes (203A, 204A) and measurement electrodes (205A, 206A) adapted to make contact to the body part to be measured and said current supplying electrodes (203A, 204A) and said measurement electrodes (205A, 206A) are mounted on a contact plate (201 A) that is abut against the body part to be measured.

13. An impedance type thickness measurement device according to claim 12 in which one pair (203A, 205A) of current supplying and measurement electrodes and another pair (204A, 206A) of current supplying and measurement electrodes are arranged on said contact plate (201A) in such manner that each pair is positioned near each side edge of the contact plate (201A) and is aligned to the other pair in the width direction of the contact plate.

14. An impedance type thickness measurement device according to any one of claims 10 to 13 in which said measurement electrodes (205; 206; 205A, 206A) are disposed between said current supplying electrodes (203, 204; 203A, 204A).

**Patentansprüche**

1. Dickenmessvorrichtung vom Impedanztyp, die umfasst:

eine Impedanzmesseinheit (104, 105, 106, 110, 112, 113, 114, 115, 116, 117, 118, 200);
eine Recheneinheit (110);
eine Speichereinheit (110, 111); und
eine Beurteilungs- und Auswertungseinheit (110), wobei die Impedanzmesseinheit (104, 105, 106, 110, 112, 113, 114, 115, 116, 117, 118, 200) geeignet ist, die Impedanz eines Körperteils, der gemessen werden soll, zu messen;
die Recheneinheit (110) geeignet ist, eine Dicke des Körperteils basierend auf der von der Impedanzmesseinheit (104, 105, 106, 110, 112, 113, 114, 115, 116, 117, 118, 200) gemessenen Impedanz zu berechnen;
die Speichereinheit (110, 111) geeignet ist, Körperbaubeurteilungs- und Auswertungsinformationen zu speichern, um die Beziehung zwischen der Dicke des Körperteils für einen Zielkörperbau und der berechneten Dicke des Körperteils darzustellen; und
die Beurteilungs- und Auswertungseinheit (110) geeignet ist, den Körperbau auf der Basis der von der Recheneinheit (110) berechneten Dicke unter Bezug auf die in der Speichereinheit (110, 111) gespeicherten Körperbaubeurteilungs- und Auswertungsinformationen zu beurteilen und auszuwerten,
**dadurch gekennzeichnet, dass** sie ferner umfasst:

eine Anzeigeeinheit (102), wobei die Anzeigeeinheit (102) geeignet ist, ein Körpermodell anzuzeigen, das aus einer Vielzahl von Körperteilen besteht, die unabhängig gemessen werden sollen, und das Ergebnis der Beurteilung und Auswertung für den Körperbau, die von der Beurteilungs- und Auswertungseinheit (110) durchgeführt werden, anzuzeigen, wobei die Anzeigeeinheit (102) geeignet ist, einen Körperteil mit der berechneten Dicke, die außerhalb eines Zieldickenbereichs ist, anzuzeigen, wie sie von der Beurteilungs- und Auswertungseinheit (110) bestimmt wird, indem der relevante Teil auf dem Körpermodell markiert wird.

2. Dickenmessvorrichtung vom Impedanztyp nach Anspruch 1, wobei die Impedanzmesseinheit (104, 105, 106, 110, 112, 113, 114, 115, 116, 117, 118, 200) eine Eingabeeinheit (104, 105, 106) für persönliche Informationen umfasst, durch welche persönliche Informationen eingegeben werden,
wobei die Speichereinheit (110, 111) geeignet ist, um die Körperbaubeurteilungs- und Auswertungsinformationen zu speichern, welche umfassen: eine Zielkörperbauauswahltabelle zum Darstellen der Beziehung zwischen den persönlichen Informationen und der Dicke des Körperteils für den Zielkörperbau; eine Abmessungsverhältnis-Berechnungsformel zum Berechnen des Abmessungsverhältnisses der berechneten Dicke des Körperteils zu der Dicke des Körperteils für den Zielkörperbau; und eine Körperbaubeurteilungs- und Auswertungstabelle zum Darstellen der Beziehung zwischen dem berechneten Abmessungsverhältnis, der Annehmbarkeit des Körperteils basierend auf dem Abmessungsverhältnis und einiger Hinweise über die Annehmbarkeit des Körperteils,
wobei die Beurteilungs- und Auswertungseinheit (110) geeignet ist, die Beurteilung und Auswertung für den Körperbau durch die folgenden Schritte auszuführen: Auswählen der Dicke des Körperteils für den Zielkörperteil entsprechend den durch die Eingabeeinheit für persönliche Informationen eingegebenen persönlichen Informationen unter Bezug auf die in der Speichereinheit (110, 111) gespeicherte Zielkörperbauauswahltabelle; Berechnen des Abmessungsverhältnisses durch Ersetzen des Zielkörperbaus durch die ausgewählte Dicke des Körperteils und der in der Speichereinheit (110, 111) gespeicherten Abmessungsverhältnis-Berechnungsformel durch die von der Recheneinheit (110) bereitgestellte berechnete Dicke des Körperteils; Beurteilen der Annehmbarkeit des von der Impedanzmesseinheit (104, 105, 106, 110, 112, 113, 114, 115, 116, 117, 118, 200) gemessenen Körperteils basierend auf dem auf diese Weise berechneten Abmessungsverhältnis unter Bezug auf die in der Speichereinheit (110, 111) gespeicherte Körperbaubeurteilungs- und Auswertungstabelle; und Auswählen einiger Hinweisinformationen abhängig von der Annehmbarkeit des Körperteils.

3. Dickenmessvorrichtung vom Impedanztyp nach Anspruch 1, wobei das Körpermodell derart ausgebildet ist, dass jeder Zielkörperaufbau abgebildet werden kann.

4. Dickenmessvorrichtung vom Impedanztyp nach einem der Ansprüche 2 bis 3, wobei die Dicke des Körperteils der Umfang des Körperteils ist.

5. Dickenmessvorrichtung vom Impedanztyp nach Anspruch 4, wobei die Abmessungsverhältnis-Berechnungsformel ist:

$$Pm = (Cm - Cs) \times 100/Cs$$

wobei Pm: Abmessungsverhältnis;
Cm: der berechnete Umfang des Körperteils; und
Cs: Umfang des Körperteils für Zielkörperbau
und;
die Beurteilungs- und Auswertungseinheit (110) geeignet ist, die Annehmbarkeit des Körperteils zu beurteilen, indem bestimmt wird, ob das von der Formel $Pm = (Cm - Cs) \times 100/Cs$ berechnete Abmessungsverhältnis innerhalb von $\pm3\%$ abweicht.

6. Dickenmessvorrichtung vom Impedanztyp nach einem der Ansprüche 2 bis 3, wobei die Dicke des Körperteils der Radius des Körperteils ist.

7. Dickenmessvorrichtung vom Impedanztyp nach Anspruch 6, wobei die Abmessungsverhältnis-Berechnungsformel ist:

$$P = (Rm - Rs) \times 100/Rs$$

wobei P: Abmessungsverhältnis;
Rm: der berechnete Radius des Körperteils; und
Rs: Radius des Körperteils für Zielkörperbau
und;
die Beurteilungs- und Auswertungseinheit (110) geeignet ist, die Annehmbarkeit des Körperteils zu beurteilen, indem bestimmt wird, ob das von der Formel $P = (Rm - Rs) \times 100/Rs$ berechnete Abmessungsverhältnis innerhalb von $\pm3\%$ abweicht.

8. Dickenmessvorrichtung vom Impedanztyp nach einem der Ansprüche 2 bis 3, wobei die Dicke des Körperteils der Durchmesser des Körperteils ist.

9. Dickenmessvorrichtung vom Impedanztyp nach Anspruch 8, wobei die Abmessungsverhältnis-Berechnungsformel ist:

$$P = (Dm - Ds) \times 100/Ds$$

wobei P: Abmessungsverhältnis;
Dm: der berechnete Durchmesser des Körperteils; und
Ds: Durchmesser des Körperteils für Zielkörperbau und;
die Beurteilungs- und Auswertungseinheit (110) geeignet ist, die Annehmbarkeit des Körperteils zu beurteilen, indem bestimmt wird, ob das von der Formel $P = (Dm - Ds) \times 100/Ds$ berechnete Abmessungsverhältnis innerhalb von $\pm3\%$ abweicht.

10. Dickenmessvorrichtung vom Impedanztyp nach einem der Ansprüche 1 bis 9, wobei die Impedanzmesseinheit (104, 105, 106, 110, 112, 113, 114, 115, 116, 117, 118, 200) Stromversorgungselektroden (203, 204) und Messelektroden (205, 206) umfasst, die geeignet sind, um den Kontakt mit dem Körperteil, der gemessen werden soll, herzustellen, und einen Hauptgürtel (201) und einen Erweiterungsgürtel (202) umfasst, die geeignet sind, um den Körperteil, der gemessen werden soll, gewickelt zu werden.

11. Dickenmessvorrichtung vom Impedanztyp nach Anspruch 10, wobei ein Paar (203, 205) von Stromversorgungs- und Messelektroden und ein anderes Paar (204, 206) von Stromversorgungs- und Messelektroden auf dem Hauptgürtel (201) in einer derartigen Weise angeordnet sind, dass jedes Paar (203, 205; 204, 206) nahe jedem Seitenrand des Hauptgürtels (201) angeordnet ist und in der Breitenrichtung des Gürtels mit dem anderen Paar ausgerichtet ist.

**12.** Dickenmessvorrichtung vom Impedanztyp nach einem der Ansprüche 1 bis 9, wobei die Impedanzmesseinheit (104, 105, 106, 110, 112, 113, 114, 115, 116, 117, 118, 200) Stromversorgungselektroden (203A, 204A) und Messelektroden (205A, 206A) umfasst, die geeignet sind, den Kontakt zu dem Körperteil, der gemessen werden soll, herzustellen, und die Stromversorgungselektroden (203A, 204A) und die Messelektroden (205A, 206A) auf einer Kontaktplatte (201A) montiert sind, die an dem Körperteil, der gemessen werden soll, anliegt.

**13.** Dickenmessvorrichtung vom Impedanztyp nach Anspruch 12, wobei ein Paar (203A, 205A) von Stromversorgungs- und Messelektroden und ein anderes Paar (204A, 206A) von Stromversorgungs- und Messelektroden auf der Kontaktplatte (201A) in einer derartigen Weise angeordnet sind, dass jedes Paar nahe jedem Seitenrand der Kontaktplatte (201A) angeordnet ist und in der Breitenrichtung der Kontaktplatte mit dem anderen Paar ausgerichtet ist.

**14.** Dickenmessvorrichtung vom Impedanztyp nach einem der Ansprüche 10 bis 13, wobei die Messelektroden (205, 206; 205A, 206A) zwischen den Stromversorgungselektroden (203, 204; 203A, 204A) angeordnet sind.

**Revendications**

**1.** Dispositif de mesure d'épaisseur du type à impédance, comprenant :

une unité de mesure d'impédance (104, 105, 106, 110, 112, 113, 114, 115, 116, 117, 118, 200) ;
une unité arithmétique (110) ;
une unité de stockage (110, 111) ; et
une unité d'évaluation et de jugement (110), dans lequel ladite unité de mesure d'impédance (104, 105, 106, 110, 112, 113, 114, 115, 116, 117, 118, 200) est adaptée pour mesurer l'impédance d'une partie d'un corps à mesurer ;
ladite unité arithmétique (110) est adaptée pour calculer une épaisseur de la partie du corps, d'après l'impédance mesurée par ladite unité de mesure d'impédance (104, 105, 106, 110, 112, 113, 114, 115, 116, 117, 118, 200) ;
ladite unité de stockage (110, 111) est adaptée pour stocker une information de jugement et d'évaluation de constitution physique pour montrer la relation entre l'épaisseur de la partie de corps pour une constitution physique cible et l'épaisseur calculée de la partie de corps ; et
ladite unité d'évaluation et de jugement (110) est adaptée pour juger est évaluer la constitution physique d'après l'épaisseur calculée par ladite unité arithmétique (110), en référence à l'information de jugement et d'évaluation de constitution physique stockée dans ladite unité de stockage (110, 111);
**caractérisé en ce qu'**il comprend en outre
une unité d'affichage (102), ladite unité d'affichage (102) étant adaptée pour afficher un modèle de corps fait d'une pluralité de parties de corps à mesurer indépendamment et afficher le résultat du jugement et de l'évaluation pour un constitution physique réalisés par ladite unité d'évaluation et de jugement (110), dans lequel ladite unité d'affichage (102) est adaptée pour afficher une partie de corps ayant l'épaisseur calculée qui est à l'extérieur d'une plage d'épaisseur cible, comme déterminée par ladite unité d'évaluation et de jugement (110), en marquant la partie concernée sur le modèle de corps.

**2.** Dispositif de mesure d'épaisseur du type à impédance selon la revendication 1 dans lequel ladite unité de mesure d'impédance (104, 105, 106, 110, 112, 113, 114, 115, 116, 117, 118, 200) inclut une unité d'entrée d'information personnelle (104, 105, 106) par laquelle une Information personnelle sont entrées,
ladite unité de stockage (110, 111) est adaptée pour stocker l'information de jugement et d'évaluation de constitution physique incluant: un tableau de sélection de constitution physique cible pour exhiber la relation entre l'information personnelle et l'épaisseur de la partie de corps pour la constitution physique cible ; une formule de calcul de rapport dimensionnel pour calculer le rapport dimensionnel de l'épaisseur calculée de la partie de corps à l'épaisseur de la partie de corps pour la constitution physique cible; et un tableau de jugement et d'évaluation de constitution physique cible pour montrer la relation entre le rapport dimensionnel calculé, l'acceptabilité de la partie de corps en fonction du rapport dimensionnel, et un certain conseil au sujet de l'acceptabilité de la partie de corps,
ladite unité d'évaluation et de jugement (110) est adaptée pour conduire le jugement et l'évaluation pour la constitution physique en suivant les étapes consistant à : sélectionner l'épaisseur de la partie de corps pour la constitution physique cible correspondant à l'information personnelle entrée par ladite unité d'entrée d'information personnelle, en référence au tableau de sélection de constitution physique cible stocké dans l'unité de stockage (110, 111); calculer le rapport dimensionnel en substituant l'épaisseur sélectionnée de la partie de corps pour la constitution physique cible et l'épaisseur calculée de la partie de corps fournie par ladite unité arithmétique (110) dans ladite formule de calcul de rapport dimensionnel stockée dans l'unité de stockage (110, 111) ; juger l'acceptabilité de la partie de corps mesurée par ladite unité de mesure d'impédance (104, 105, 106, 110, 112, 113, 114, 115, 116, 117, 118, 200), d'après le rapport dimensionnel ainsi calculé, en référence au tableau de jugement et d'évaluation de constitution physique stocké dans l'unité de stockage (110, 111) ; et sélectionner une certaine information de conseil selon l'acceptabilité de la partie de corps.

3. Dispositif de mesure d'épaisseur du type à impédance selon la revendication 1 dans lequel ledit modèle de corps est formé de telle manière que chaque constitution physique cible peut être mise sous forme d'image.

4. Dispositif de mesure d'épaisseur du type à impédance selon l'une quelconque des revendications 2 à 3 dans lequel l'épaisseur de la partie de corps est la circonférence de la partie de corps.

5. Dispositif de mesure d'épaisseur du type à impédance selon la revendication 4 dans lequel ladite formule de calcul de rapport dimensionnel est :

$$Pm = (Cm - Cs) \times 100/Cs$$

où Pm : rapport dimensionnel
Cm : circonférence calculée de la partie de corps
Cs : circonférence de la partie de corps pour la constitution physique cible
et ;
ladite unité d'évaluation et de jugement (110) est adaptée pour juger l'acceptabilité de la partie de corps en déterminant si le rapport dimensionnel calculé par ladite formule $Pm = (Cm - Cs) \times 100/Cs$ a dévié de $\pm$ 3%.

6. Dispositif de mesure d'épaisseur du type à impédance selon l'une quelconque des revendications 2 à 3 dans lequel l'épaisseur de la partie de corps est le rayon de la partie de corps.

7. Dispositif de mesure d'épaisseur du type à impédance selon la revendication 6 dans lequel ladite formule de calcul de rapport dimensionnel est :

$$P = (Rm - Rs) \times 100/Rs$$

où P : rapport dimensionnel
Rm : rayon calculé de la partie de corps
Rs : rayon de la partie de corps pour la constitution physique cible
et ;
ladite unité d'évaluation et de jugement (110) est adaptée pour juger l'acceptabilité de la partie de corps en déterminant si le rapport dimensionnel calculé par ladite formule $P = (Rm - Rs) \times 100/Rs$ a dévié de $\pm$ 3%.

8. Dispositif de mesure d'épaisseur du type à impédance selon l'une quelconque des revendications 2 à 3 dans lequel l'épaisseur de la partie de corps est le diamètre de la partie de corps.

9. Dispositif de mesure d'épaisseur du type à impédance selon la revendication 8 dans lequel ladite formule de calcul de rapport dimensionnel est :

$$P = (Dm - Ds) \times 100/Ds$$

où P : rapport dimensionnel
Dm : diamètre calculé de la partie de corps
Ds : diamètre de la partie de corps pour la constitution physique cible
et;
ladite unité d'évaluation et de jugement (110) est adaptée pour juger l'acceptabilité de la partie de corps en déterminant si le rapport dimensionnel calculé par ladite formule $P = (Dm - Ds) \times 100/Ds$ a dévié de $\pm$ 3%.

10. Dispositif de mesure d'épaisseur du type à impédance selon l'une quelconque des revendications 1 à 9 dans lequel ladite unité de mesure d'impédance (104, 105, 106, 110, 112, 113, 114, 115, 116, 117, 118, 200) inclut des électrodes d'alimentation en courant (203, 204) et des électrodes de mesure (205, 206) adaptées pour entrer en contact avec la partie de corps à mesurer et comprend une sangle principale (201) et une sangle d'extension (202) qui sont adaptées pour être enroulée autour de la partie de corps à mesurer.

11. Dispositif de mesure d'épaisseur du type à impédance selon la revendication 10 dans lequel une paire (203, 205) d'électrodes de mesure et d'alimentation en courant et une autre paire (204, 206) d'électrodes de mesure et d'alimentation en courant sont agencées sur ladite sangle principale (201) de telle manière que chaque paire (203, 205; 204, 206) est positionnée près de chaque bord de la sangle principale (201) et est alignée avec l'autre paire dans la direction de la largeur de la sangle.

12. Dispositif de mesure d'épaisseur du type à Impédance selon l'une quelconque des revendications 1 à 9 dans lequel ladite unité de mesure d'impédance (104, 105, 106, 110, 112, 113, 114, 115, 116, 117, 118, 200) inclut des électrodes d'alimentation en courant (203A, 204A) et des électrodes de mesure (205A, 206A) adaptées pour entrer en contact avec la partie de corps à mesurer et lesdites électrodes d'alimentation en courant (203A, 204A) et lesdites électrodes de mesure (205A, 206A) sont montées sur une plaque de contact (201A) qui est en butée contre la partie de corps à mesurer.

13. Dispositif de mesure d'épaisseur du type à impédance selon la revendication 12 dans lequel une paire (203A, 205A) d'électrodes de mesure et d'alimentation en courant et une autre paire (204A, 206A) d'électrodes de mesure et d'alimentation en courant sont agencées sur ladite plaque de contact (201A) de telle manière que chaque paire est positionnée près de chaque bord latéral de la plaque de contact (201A) et est alignée avec l'autre paire dans la direction de la largeur de la plaque de contact.

14. Dispositif de mesure d'épaisseur du type à impédance selon l'une quelconque des revendications 10 à 13 dans lequel lesdites électrodes de mesure (205, 206; 205A, 206A) sont disposées entre lesdites électrodes d'alimentation en courant (203, 204; 203A, 204A).

# FIG. 1

REGION OVER WHICH GIRTH
IS MEASURED (Belt Width)

200

201

203

205

206

204

INNER
SURFACE

OUTER
SURFACE

202

1

101

102

100

107

104

SETTING

105

103

106

ON
OFF

# FIG. 2

103, 104, 105, 106

DISPLAY UNIT — 102

SWITCHES
( ON/OFF KEY
UP KEY
DOWN KEY
SETTING KEY )

EXTERNAL INPUT/OUTPUT INTERFACE
( INTERFACE CIRCUIT 119
CONNECTOR 107 )

107, 119

MICROCOMPUTER — 110

AUXILIARY STORAGE UNIT — 111

FILTER CIRCUIT — 112

AC CURRENT OUTPUT CIRCUIT — 113

114: REFERENCE RESISTOR

203, 204: CURRENT SUPPLYING ELECTRODE

A/D CONVERTER — 118

SWITCHING UNIT — 117

DIFFERENTIAL AMPLIFIER — 115

DIFFERENTIAL AMPLIFIER — 116

205, 206: MEASUREMENT ELECTRODE

EP 1 514 514 B1

# FIG. 3

200:DETECTOR

RIGHT FRONT ARM

LEFT FRONT ARM

RIGHT UPPER ARM

LEFT UPPER ARM

1:CORD

TRUNK PORTION

RIGHT UPPER LEG

LEFT UPPER LEG

LEFT LOWER LEG

100:MAIN FRAME

RIGHT LOWER LEG

18

# FIG. 4

START

ENTER PERSONAL INFORMATION — S1

ENTER TARGET BODY BUILD — S2

MEASURE IMPEDANCE ON EACH BODY PART — S3

CALCULATE GIRTH OF EACH BODY PART — S4

JUDGE AND ASSESS BODY BUILD — S5

DISPLAY ADVICE MESSAGE — S6

END

# FIG. 5

```
    ┌─────────────────────────────────┐
    │   JUDGE AND ASSESS BODY BUILD   │
    └─────────────────────────────────┘
                    │
                    ▼
    ┌─────────────────────────────────┐
    │ CALCULATE DIMENSION RATE OF GIRTH│
    │ OF BODY PART TO GIRTH FOR TARGET │─── S51
    │ BODY BUILD                       │
    └─────────────────────────────────┘
                    │
                    ▼
    ┌─────────────────────────────────┐
    │      JUDGE BALANCE OF LIMBS      │─── S52
    └─────────────────────────────────┘
                    │
                    ▼
    ┌─────────────────────────────────┐
    │       JUDGE TRUNK PORTION        │─── S53
    └─────────────────────────────────┘
                    │
                    ▼
    ┌─────────────────────────────────┐
    │      SELECT ADVICE MESSAGE       │
    │      (ASSESS BODY BUILD)         │─── S54
    └─────────────────────────────────┘
                    │
                    ▼
    ┌─────────────────────────────────┐
    │             RETURN              │
    └─────────────────────────────────┘
```

# FIG. 6

| JUDGE ACCEPTABILITY OF BODY PART | | | ADVICE |
|---|---|---|---|
| -3% ≤ DIMENSION RATE ≤ +3% | FOUR LIMBS AND TRUNK PORTION | | CONGRATULATION! YOU HAVE TARGET BODY BUILD. MAINTAIN SUCH BODY BUILD. |
| +3% < DIMENSION RATE | LIMBS | ONLY A RIGHT UPPER LIMB | LITTLE MORE TIGHTEN ONLY A RIGHT UPPER LIMB WITH E.G. AN EXERCISE USING A DUMBBELL TO GET MORE BALANCED BODY BUILD! |
| | | ONLY A LEFT UPPER LIMB | LITTLE MORE TIGHTEN ONLY A LEFT UPPER LIMB WITH E.G. AN EXERCISE USING A DUMBBELL TO GET MORE BALANCED BODY BUILD! |
| | | ONLY A RIGHT LOWER LIMB | LITTLE MORE TIGHTEN ONLY A RIGHT LOWER LIMB WITH E.G. AN EXPANSION AND CONTRACTION EXERCISE TO GET MORE BALANCED BODY BUILD! |
| | | ONLY A LEFT LOWER LIMB | LITTLE MORE TIGHTEN ONLY A LEFT LOWER LIMB WITH E.G. AN EXPANSION AND CONTRACTION EXERCISE TO GET MORE BALANCED BODY BUILD! |
| | | RIGHT UPPER AND LEFT UPPER LIMBS | TIGHTEN UPPER LIMBS WITH E.G. A PUSH-UP EXERCISE TO GET MORE BALANCED BODY BUILD! |
| | | RIGHT LOWER AND LEFT LOWER LIMBS | TIGHTEN LOWER LIMBS WITH E.G. A WALKING EXERCISE TO GET MORE BALANCED BODY BUILD! |
| | | RIGHT UPPER AND RIGHT LOWER LIMBS | TIGHTEN RIGHT UPPER AND RIGHT LOWER LIMBS WITH E.G. AN EXERCISE USING A DUMBBELL AND AN EXPANSION AND CONTRACTION EXERCISE TO GET MORE BALANCED BODY BUILD! |
| | | LEFT UPPER AND LEFT LOWER LIMBS | TIGHTEN LEFT UPPER AND LEFT LOWER LIMBS WITH E.G. AN EXERCISE USING A DUMBBELL AND AN EXPANSION AND CONTRACTION EXERCISE TO GET MORE BALANCED BODY BUILD! |
| | | RIGHT UPPER AND LEFT LOWER LIMBS | TIGHTEN RIGHT UPPER AND LEFT LOWER LIMBS WITH E.G. AN EXERCISE USING A DUMBBELL AND AN EXPANSION AND CONTRACTION EXERCISE TO GET MORE BALANCED BODY BUILD! |
| | | LEFT UPPER AND RIGHT LOWER LIMBS | TIGHTEN LEFT UPPER AND RIGHT LOWER LIMBS WITH E.G. AN EXERCISE USING A DUMBBELL AND AN EXPANSION AND CONTRACTION EXERCISE TO GET MORE BALANCED BODY BUILD! |
| | | OTHERS | PAY EFFORT LITTLE MORE! |
| | TRUNK PORTION | | TIGHTEN TRUNK PORTION WITH E.G. ABDOMINAL MUSCLE AND BACK MUSCLE EXERCISE! |
| DIMENSION RATE < -3% | LIMBS | ONLY A RIGHT UPPER LIMB | HAVE ENOUGH MEAL AND TRAIN ONLY A RIGHT UPPER LIMB WITH E.G. AN EXERCISE USING A DUMBBELL TO GET MORE BALANCED BODY BUILD! |
| | | ONLY A LEFT UPPER LIMB | HAVE ENOUGH MEAL AND TRAIN ONLY A LEFT UPPER LIMB WITH E.G. AN EXERCISE USING A DUMBBELL TO GET MORE BALANCED BODY BUILD! |
| | | ONLY A RIGHT LOWER LIMB | HAVE ENOUGH MEAL AND TRAIN ONLY A RIGHT LOWER LIMB WITH E.G. AN EXPANSION AND CONTRACTION EXERCISE TO GET MORE BALANCED BODY BUILD! |
| | | ONLY A LEFT LOWER LIMB | HAVE ENOUGH MEAL AND TRAIN ONLY A LEFT LOWER LIMB WITH E.G. AN EXPANSION AND CONTRACTION EXERCISE TO GET MORE BALANCED BODY BUILD! |
| | | RIGHT UPPER AND LEFT UPPER LIMBS | HAVE ENOUGH MEAL AND TIGHTEN UPPER LIMBS WITH E.G. A PUSH-UP EXERCISE TO GET MORE BALANCED BODY BUILD! |
| | | RIGHT LOWER AND LEFT LOWER LIMBS | HAVE ENOUGH MEAL AND TIGHTEN LOWER LIMBS WITH E.G. A WALKING EXERCISE TO GET MORE BALANCED BODY BUILD! |
| | | RIGHT UPPER AND RIGHT LOWER LIMBS | HAVE ENOUGH MEAL AND TRAIN RIGHT UPPER AND RIGHT LOWER LIMBS WITH E.G. AN EXERCISE USING A DUMBBELL AND AN EXPANSION AND CONTRACTION EXERCISE TO GET MORE BALANCED BODY BUILD! |
| | | LEFT UPPER AND LEFT LOWER LIMBS | HAVE ENOUGH MEAL AND TRAIN LEFT UPPER AND LEFT LOWER LIMBS WITH E.G. AN EXERCISE USING A DUMBBELL AND AN EXPANSION AND CONTRACTION EXERCISE TO GET MORE BALANCED BODY BUILD! |
| | | RIGHT UPPER AND LEFT LOWER LIMBS | HAVE ENOUGH MEAL AND TRAIN RIGHT UPPER AND LEFT LOWER LIMBS WITH E.G. AN EXERCISE USING A DUMBBELL AND AN EXPANSION AND CONTRACTION EXERCISE TO GET MORE BALANCED BODY BUILD! |
| | | LEFT UPPER AND RIGHT LOWER LIMBS | HAVE ENOUGH MEAL AND TRAIN LEFT UPPER AND RIGHT LOWER LIMBS WITH E.G. AN EXERCISE USING A DUMBBELL AND AN EXPANSION AND CONTRACTION EXERCISE TO GET MORE BALANCED BODY BUILD! |
| | | OTHERS | PAY EFFORT LITTLE MORE! |
| | TRUNK PORTION | | HAVE ENOUGH MEAL AND TRAIN TRUNK PORTION WITH E.G ABDOMINAL MUSCLE AND BACK MUSCLE EXERCISE! |

# FIG. 7

★★★ ENTER DATA ★★★

■ AGE:            20   YEARS OLD

■ SEX:            MALE

■ HEIGHT:         160 cm

■ BODY WEIGHT:    55 kg

CURSOR

EP 1 514 514 B1

# FIG. 8

SELECTION

☆☆☆ SELECT ONE FOR TARGET BODY BUILD ☆☆☆

☑ "MODEL" TYPE BODY BUILD ☐ "STANDARD" TYPE BODY BUILD ☐ "ATHLETE" TYPE BODY BUILD

| | MODEL | STANDARD | ATHLETE |
|---|---|---|---|
| RIGHT FRONT ARM: | 24cm | 25cm | 28cm |
| LEFT FRONT ARM: | 24cm | 25cm | 28cm |
| RIGHT UPPER ARM: | 26cm | 27cm | 31cm |
| LEFT UPPER ARM: | 26cm | 27cm | 31cm |
| TRUNK PORTION: | 74cm | 77cm | 81cm |
| RIGHT UPPER LEG: | 48cm | 51cm | 56cm |
| LEFT UPPER LEG: | 48cm | 51cm | 56cm |
| RIGHT LOWER LEG: | 36cm | 40cm | 45cm |
| LEFT LOWER LEG: | 36cm | 40cm | 45cm |

BODY MODEL

IDEAL VALUE

EP 1 514 514 B1

# FIG. 9

☆☆☆ FOLLOWING INPUT ITEMS ARE OK? ☆☆☆

TARGET:  "MODEL"  TYPE BODY BUILD

■ AGE:  23 YEARS OLD

■ SEX:        MALE

■ HEIGHT:      175 cm

■ BODY WEIGHT: 74 kg

RIGHT FRONT ARM:  24cm
LEFT FRONT ARM:  24cm
RIGHT UPPER ARM:  26cm
LEFT UPPER ARM:  26cm
TRUNK PORTION:  74cm
RIGHT UPPER LEG:  48cm
LEFT UPPER LEG:  48cm
RIGHT LOWER LEG:  36cm
LEFT LOWER LEG:  36cm

MEASUREMENT START

RETURNS TO INPUT SCREEN

CURSOR

A SHADED PART MEANS A BODY
PART TO BE MEASURED

# FIG. 10

EP 1 514 514 B1

☆☆☆ FOLLOWINGS ARE YOUR MEASUREMENT RESULT. ☆☆☆

**MEASUREMENT RESULT**

RIGHT FRONT ARM: 24cm
LEFT FRONT ARM: 24cm
RIGHT UPPER ARM: 26cm
LEFT UPPER ARM: 26cm
TRUNK PORTION: 77cm
RIGHT UPPER LEG: 51cm
LEFT UPPER LEG: 49cm
RIGHT LOWER LEG: 39cm
LEFT LOWER LEG: 37cm

**TARGET**

RIGHT FRONT ARM: 24cm
LEFT FRONT ARM: 24cm
RIGHT UPPER ARM: 26cm
LEFT UPPER ARM: 26cm
TRUNK PORTION: 74cm
RIGHT UPPER LEG: 48cm
LEFT UPPER LEG: 48cm
RIGHT LOWER LEG: 36cm
LEFT LOWER LEG: 36cm

LITTLE MORE TIGHTEN ONLY A RIGHT LOWER LIMB WITH E. G. AN EXPANSION AND CONTRACTION
EXERCISE TO GET MORE BALANCED BODY BUILD !
TIGHTEN TRUNK PORTION WITH E. G ABDOMINAL MUSCLE AND BACK MUSCLE EXERCISE !

# FIG. 11

# FIG. 12

☆☆☆ SELECT ONE FOR TARGET BODY BUILD ☆☆☆

☑ "MODEL" TYPE BODY BUILD    ☐ "STANDARD" TYPE BODY BUILD    ☐ "ATHLETE" TYPE BODY BUILD

RIGHT ARM: 25cm
LEFT ARM: 25cm
TRUNK PORTION: 74cm
RIGHT UPPER LEG: 48cm
LEFT UPPER LEG: 48cm

RIGHT ARM: 26cm
LEFT ARM: 26cm
TRUNK PORTION: 77cm
RIGHT UPPER LEG: 51cm
LEFT UPPER LEG: 51cm

RIGHT ARM: 29cm
LEFT ARM: 29cm
TRUNK PORTION: 81cm
RIGHT UPPER LEG: 56cm
LEFT UPPER LEG: 56cm

EP 1 514 514 B1

# FIG. 13

☆☆☆ FOLLOWING INPUT ITEMS ARE OK ? ☆☆☆

TARGET: "MODEL" TYPE BODY BUILD

■ AGE: 23 YEARS OLD

■ SEX: MALE

■ HEIGHT: 175 cm

■ BODY WEIGHT: 74 kg

RIGHT ARM: 25cm
LEFT ARM: 25cm
TRUNK PORTION: 74cm
RIGHT UPPER LEG: 48cm
LEFT UPPER LEG: 48cm

MEASUREMENT START        RETURNS TO INPUT SCREEN

EP 1 514 514 B1

# FIG. 14

EP 1 514 514 B1

☆☆☆ FOLLOWINGS ARE YOUR MEASUREMENT RESULT. ☆☆☆

MEASUREMENT RESULT

TARGET

RIGHT ARM: 27cm
LEFT ARM: 25cm
TRUNK PORTION: 77cm
RIGHT UPPER LEG: 51cm
LEFT UPPER LEG: 50cm

RIGHT ARM: 25cm
LEFT ARM: 25cm
TRUNK PORTION: 74cm
RIGHT UPPER LEG: 48cm
LEFT UPPER LEG: 48cm

PAY EFFORT LITTLE MORE! TIGHTEN TRUNK PORTION WITH E.G. ABDOMINAL MUSCLE AND BACK MUSCLE EXERCISE !

# FIG. 15

205A

206A

203A

201A

200A

202A

204A

# FIG. 16

205B

206B

203B

200B

204B

# FIG. 17

| | | ⋯ | 150cm～ | 160cm～ | 170cm～ | ⋯ |
|---|---|---|---|---|---|---|
| Male | 10's | ⋮ | ⋮ | ⋮ | ⋮ | ⋮ |
| | 20's | ⋯ | ⋯ | FOR "MODEL" TYPE BODY BUILD<br><br>RIGHT FRONT ARM: 24cm<br>LEFT FRONT ARM: 24cm<br>RIGHT UPPER ARM: 26cm<br>LEFT UPPER ARM: 26cm<br>TRUNK PORTION: 74cm<br>RIGHT UPPER LEG: 48cm<br>LEFT UPPER LEG: 48cm<br>RIGHT LOWER LEG: 36cm<br>LEFT LOWER LEG: 36cm<br><br>FOR "STANDARD" TYPE BODY BUILD<br><br>RIGHT FRONT ARM: 25cm<br>LEFT FRONT ARM: 25cm<br>RIGHT UPPER ARM: 27cm<br>LEFT UPPER ARM: 27cm<br>TRUNK PORTION: 77cm<br>RIGHT UPPER LEG: 51cm<br>LEFT UPPER LEG: 51cm<br>RIGHT LOWER LEG: 40cm<br>LEFT LOWER LEG: 40cm<br><br>FOR "ATHLETE" TYPE BODY BUILD<br><br>RIGHT FRONT ARM: 28cm<br>LEFT FRONT ARM: 28cm<br>RIGHT UPPER ARM: 31cm<br>LEFT UPPER ARM: 31cm<br>TRUNK PORTION: 81cm<br>RIGHT UPPER LEG: 56cm<br>LEFT UPPER LEG: 56cm<br>RIGHT LOWER LEG: 45cm<br>LEFT LOWER LEG: 45cm | ⋯ | ⋯ |
| | 30's | ⋮ | ⋮ | ⋮ | ⋮ | ⋮ |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000350727 A
- JP 2002191563 A
- WO 01076485 A
- JP 2001 A

- JP 178696 A
- JP 2001178696 A
- EP 1329191 A1
- US 5184624 A